# EUROPEAN PATENT APPLICATION

(11) **EP 0 844 002 A1**
(43) Date of publication of application: **27.05.1998**
(21) Application number: 97119720.7
(22) Date of filing: 11.11.1997
(51) Int. Cl.: A61K 31/715

(54) **Use of activated hemicellulose for the induction of interleukin-12**

(30) Priority: 11.11.1996 JP 315498/96
(71) Applicant: Yagita, Akikuni, Mitaki-shi, Tokyo 181 (JP)
(72) Inventor: Yagita, Akikuni, Mitaki-shi, Tokyo 181 (JP)
(74) Representative: VOSSIUS & PARTNER

(57) **Abstract**

A pharmaceutical composition comprising activated hemicellulose, use of activated hemicellulose for the preparation of a pharmaceutical composition for inducing IL-12, and use of activated hemicellulose for the preparation of a pharmaceutical composition for treating cancer are provided.

## Description

The present invention relates to a substance that can induce interleukin-12 in vivo, and to a pharmaceutical composition comprising the same.

Interleukin-12 (IL-12) was originally found to be a cytokine that can activate NK cells. Subsequent studies revealed that it also promotes growth of and activates cytotoxic T cells which are directed against tumor cells (killer T cells), and further promotes the production of interferon γ (IFN γ) that stimulates activation of killer T cells. Thus, IL-12 has attracted attention as a substance effective in the treatment of human cancer patients.

Recently, large-scale production of IL-12 by using gene manipulation techniques was realized in the U.S. (Recombinant IL-12 (rt-IL-12)). Following that, a method for directly introducing the IL-12 producing gene into tumor cells has been developed by using gene introduction techniques in order to directly deliver IL-12 to cancer cells.

However, sensitivity of rt-IL-12 used in the method is low and a large-amount administration is needed, for example, for treating human cancer. Such large-amount administrations cause various side effects including fever and inappetence. Besides these serious side effects, several problems regarding this method are pointed out, that is, e.g., the gene manipulation procedure is so complicated that it needs much labor and lacks economical feasibility.

For using IL-12 to stop tumor growth or make it disappear, there are two methods; one is to externally administer IL-12, and the other is to induce in vivo IL-12 production from the endogenous gene. Such IL-12 production induced in vivo does not raise an abnormal immune response, thereby resolving intrinsical problems of rt-IL-12. At the same time, said IL-12 induced in vivo is so highly sensitive that an extensive tumor loss effect can be expected. However, a substance that can efficiently induce IL-12 production from the endogenous gene is not known.

Thus, the technical problem underlying the present invention is to provide a method for inducing IL-12 in vivo in a tumor bearing patient and a substance having such an induction effect, and further to provide pharmaceutical compositions comprising said substance.

The solution to the above technical problem is achieved by providing the embodiments characterized in the claims.

Accordingly, the present invention relates to an interleukin-12 inducer, characterized by containing activated hemicellulose (AHCC) or derivatives thereof and a medical composition comprising the same.

The Activated Hemi Cellulose Compound (AHCC) of the present invention is known as a biologically active substance which is obtained by enzyme treatment of a plant fiber existing in the cell wall of mycelia of fungi, and contains heteroglucans including β-(1-3)D-glucan, β-(1-6)D-glucan and α-(1-4)D-glucan, peptidoglucan, proteoglucan, lectin, nucleic acids and indigestive polysaccharides.

The IL-12 inducer of the present invention may consist only of the above described AHCC or derivatives thereof, but preferably further contains components of fungal mycelia (Eubasidomycetes). Preferably, said components are derived from mushrooms. Examples of such components of fungal mycelia include PSK, which is a mycelian component of a polypore (*Polyporacede*) and is used as an anti-cancer agent, SPG, which is a mycelian component of *Schizophyllum commune*, and lentinan, which is a mycelian component of a shiitake *(Lentinula edodes)*, but are not limited thereto.

Further, such components of fungal mycelia include, for example, that of agarisku, *Ganoderma lucidum,* ningyotake, kawariharatake, niousimeji, kabenoanatake, *Griforia frondosa, Hericium erinaceum*, an oyster fungus (*Pleurotus ostreatus*), mannnenntake (*Ganoderma lucidum*), *Panellus serotinus*, kobutake, *Lenzites betulina*, a matsu-take (*Tricholoma matsutake)*, bekkoutake, nametake and *Frammulina velutipes*.

The IL-12 inducer of the present invention is preferably composed of only the above described AHCC or derivatives thereof and the components of said fungal mycelia, but more preferably additionally contains bacterial components of hemolytic streptococci. Examples of such bacterial components of hemolytic streptococci include OK-432, which is a known anti-cancer agent, but are not limited thereto.

These are substances known as biological response modifiers (BRMs).

It has been revealed that in progressed cancer or end-stage cancer, tumor regression or disappearance could not be obtained by established treatments in modern medicine such as surgery, administration of anti-cancer agents, radiotherapy or hormone therapy. On the other hand, the IL-12 inducer of the present invention is effective in causing tumor regression and disappearance in patients with progressed or end-stage cancer.

No side effects were observed when the IL-12 inducer of the present invention was administered. Rt-IL-12 produced by the conventional gene manipulation method has low sensitivity and therefore requires to be administered in large doses, which often induces various side effects and exerts serious damages in patients. On the other hand, the IL-12 inducer of the present invention does not induce any side effects because it stimulates the IL-12 production from the endogenous gene and, therefore, it is not necessary to externally administer a substance causing side effects.

The medical composition of the present invention comprises the above described IL-12 inducer as the main component. The medical composition of the present invention can be used, for example, as an anti-cancer agent but is not limited thereto.

The formulation of the medical composition of the present invention to be administered to a human being or to an animal is not specifically limited. The medical composition of the present invention may be, for example, retained by carriers and suitably prepared into various formulations commonly used in medicine.

As carrier, at least one selected from solid, semi-solid or liquid diluent, filler or other support for formulation may be used at a ratio of, for example, 0.1% to 99.5%, preferably 0.5% to 90%. The medical composition of the present invention may be safely administered orally or parenterally. The route of parenteral administration includes local administration such as intratissue administration, subcutaneous administration, intramuscular administration, intra arterial/intravenous administration and per rectum administration. Formulations suitable for the above administration routes are well known in the art.

For example, when to be used as an anti-cancer agent, the dosage is preferably determined according to age, body weight, administration route used, type of disease and severity. In the case of human patients, it is generally administered orally at a dosage of 100 to 20,000 mg active compound /day, preferably 1,000 to 10,000 mg/day. For parenteral administration, the dosage largely differs according to the administration route, but in general, 100 to 1,000 mg/day, preferably 200 to 500 mg/day may be used. According to each case, a lower dosage may be enough, or a larger dosage may be required. The dosage can be divided into two to four doses per day.

For oral administration, a solid or liquid formulation may be used such as pulvis, powder, granules, tablets, capsules, syrup, an elixir or a suspension.

Pulvis is prepared by pulverizing the active substance to a suitable fineness. Powder is prepared by pulverizing the active substance to a suitable fineness, and then mixing it with a medical carrier prepared to the same fineness, for example, dietary carbohydrates such as starch and mannitol, or other vehicles. If necessary, a corrective, preservatives, a dispersing agent, tinctions, aromatics and/or others may be added thereto.

Capsules are prepared by filling the above described pulverized pulvis, powder or granulated tablets into an outer capsule, for example, a gelatine capsule. Before filling, a lubricant or drifting agent, for example, colloidal silica, talc, magnesium stearate, calcium stearate or solid polyethylene glycol may be added. The efficacy of a medicine in capsule formulation after intake is improved by adding thereto a disintegrator or solubilizing agent such as carboxymethyl cellulose, calcium carboxymethyl cellulose, low-substituted hydroxypropyl cellulose, sodium crosskarumelose, sodium carboxystarch, calcium carbonate or sodium carbonate.

Soft capsules may be prepared by suspending fine powder of the product of the present invention in plant oil, polyethylene glycol, glycerin, or a surfactant and encapsulate them within a gelatin sheet.

Granules may be prepared by mixing the pulverized active substance and the above described vehicle, a disintegrator and, if necessary, a binder (for example, sodium carboxymethyl cellulose, hydroxypropyl cellulose, methyl cellulose, hydroxypropylmethyl cellulose, gelatin, polyvinylpyrrolidone, polyvinylalcohol, and the like), or a humectant (for example, syrup, starch, gum arabic, cellulose solution, macromolecule solution, and the like), kneading them, and letting them pass through a sieve. Alternatively to granulate powders, granules may be obtained by pulverizing slugs of insufficient shapes which had appeared at tableting. A dissolution delaying agent (for example, paraffin, wax, hardened castor oil, and the like), a reabsorption agent (for example, quaternary salts and the like) or an adsorbent (for example, bentonite, Kaolin, dicalcium phosphate, and the like) may be mixed therewith in advance.

Tablets may be prepared by adding a lubricant including stearic acid, stearate, talc, mineral oil, and the like to thus obtained granules, and then tableting. Thus obtained, intact tablets may be covered with a film coating or sugar coating.

The mixing process of the active substance of the present invention and a fluid inactive carrier may be immediately followed by the tableting process without the above described granulation process or slug process. Coatings that can be used include a transparent or semi-transparent protective coating of a closed coating of shellac, a coating of sugars or macromolecules and a finish coating consisting of wax.

Other oral formulations, including syrup, an elixir and a suspension can be prepared in a dosage per unit so that a predetermined amount of the medical substance is contained in a predetermined amount of each formulation. Syrup may be prepared by dissolving the active agent in a suitable flavored water solution. An elixir may be prepared by using a non-toxic alcoholic carrier. A suspension may be prepared by dispersing the active substance in non-toxic carriers. A suspending agent or emulsifier (for example, ethoxylated isostearyl alcohols or polyoxyethylene sorbitol esters), a preservative, a corrective (for example, peppermint oil or saccharin) and other agents may be added thereto.

If necessary, the dosage unit for oral administration may be contained in microcapsules. In this method, the active substance is coated or buried in macromolecules or wax, thereby prolonging action time or delaying the release.

Subcutaneous, intramuscular, intra-arterial and intravenous administration can be given by using liquid dosage unit formulations, for example, an injection consisting of a solution or suspension. These formulations are prepared by dissolving or suspending a predetermined amount of the active substance in a non-toxic liquid carrier suitable for an objective injection route including aqueous or oleaginous solvents and then sterilizing the solution or suspension. Alternatively, a predetermined amount of the powder or the freeze-dried active substance may be placed in a vial and the vial and contents thereof may be sterilized and sealed. In this case, the active substance is to be dissolved or mixed immediately before the administration, and spare vials or carriers can be reserved. Non-toxic salts or salt solutions to prepare an isotonic injection may be added and further a stabilizer, preservative, suspending agent, emulsifier and/or others may be combinedly used.

The formulation for per rectal administration may be prepared by mixing the active substance with hydrophobic or hydrophilic suppository base including, for example, polyethylene glycol, cacao butter, higher esters (for example, the myristyl ester of palmitic acid) and mixtures thereof.

AHCC, components of fungal mycelia, and bacterial components of hemolytic streptococci of the present invention have the effect of inducing IL-12. Accordingly, a method for inducing IL-12 in vivo by administering these substances is within the scope of the present invention.

Further, AHCC, components of fungal mycelia, and bacterial components of hemolytic streptococci of the present invention have the effect of inducing IL-12. Accordingly, a method for curing cancer by administering them in single or multiple administrations at a dose that can induce IL-12 is within the scope of the present invention.

The present invention is illustrated by the following examples.

### Example 1: Administration of AHCC only

A human patient suffering from esophagus cancer (72-year-old male) showing infiltration at the cervical and intraperitoneal lymph nodes with no indication for surgery, being unresponsive to radiotherapy, and unable to oral intake of other than water was treated with AHCC.

To this patient, AHCC was continuously administered at a dosage of 3.0 g/day. One month after the start of the administration, the patient became able to eat 30% gruel or 50% gruel. The level of serum SCC tumor marker was 23 ng/mL before the administration (normal level: 2.0 ng/mL), but improved to 1.3 ng/mL one month later. By further continuing the administration, he became able to eat 100% gruel three month after the administration start, when the tumor was confirmed to have completely disappeared (complete regression [CR]) by fluoroscopic and CT examinations of the esophagus.

After a three-month administration of AHCC, serum NK activity was as high as 68% (normal value: lower than 40%), and serum IL-12 level was as high as 78 pg/mL (normal value: lower than 7.2 pg/mL). These result are listed in Table 1.

In Table 1, CD4 represents helper T cells, and CD8 represents killer T cells. The CD4/CD8 ratio represents the degree of increase in killer T cells, and a value less than 1 represents an increase in killer T cells. The range of the normal value is 1.0 to 1.5. The CD4/CD8 ratio in Example 1 was 0.75, showing that the level of cytotoxic killer T cells increased.

### Example 2: Administration of AHCC only

In a human patient suffering from orchioncus (36-year-old male), primary cancer in the right testis was excised. After the surgery, a metastasis of an infant's head size occurred at a peritoneal lymph node. AHCC at a dosage of 3.0 g/day was continuously administered orally to this patient. After one month, the size of tumor decreased approximately by half, and after three month the tumor completely disappeared.

After a three month administration, NK activity was low as 13%, but the IL-12 level was as high as 120 pg/mL. It was suggested that the anti-tumor effect of AHCC was not accomplished through the NK activity. These results are listed Table 1.

**Table 1**

| | Human patient | Symptoms | NK Activity | Serum IL-12 level | Treatment evaluation | CD4/CD8 ratio |
|---|---|---|---|---|---|---|
| Example 1 | 72-year-old male | esophagus cancer; surgery impossible; radiotherapy unresponsive; end-stage cancer | 68% | 78 ng/mL | CR; complete regression | 0.75 |
| Example 2 | 36-year-old male | right testis tumor; infant's head size metastasis to retroperitoneal lymph node; end-stage cancer | 13% | 120 ng/mL | CR; complete regression | 0.52 |

### Examples 3 to 6: Combined administration of AHCC with shark cartilage

To the human cancer patients listed in Table 2, AHCC at a dosage of 3.0 g/day and concomitantly shark cartilage (β shark) at 20 g/day were continuously administered orally. After a three month administration, treatment evaluation and NK Activity, IL-12 level and the CD4/CD8 ratio were evaluated. According to Examples 3 and 6, tumor regression of 50% (partial regression [PR]) or more was obtained in either case. However, the NK activity in either example was within the normal range, indicating that the NK activity was not elevated. In either of Examples 3 to 6, the IL-12 level was high, significantly exceeding the normal range. It was suggested that IL-12 was involved in the tumor regression. Further, from the results of previous immunological investigations, it has been shown that concomitant use of shark cartilage had no effect on the NK activity and did not elevate the IL-12 level.

**Table 2**

| | Human patient | Symptoms | NK activity | Serum IL-12 level | Treatment evaluation | CD4/CD8 ratio |
|---|---|---|---|---|---|---|
| Example 3 | 72-year-old male | stomach cancer; progressed stage | 24% | 240 ng/mL | CR; complete regression | 0.34 |
| Example 4 | 57-year-old male | caecum cancer; carcinomatous peritonitis; end-stage cancer | 62% | 230 ng/mL | CR; complete regression | 0.42 |
| Example 5 | 69-year-old male | stomach cancer; metastasis to liver; end-stage cancer | 62% | 103 ng/mL | CR; complete regression | 0.62 |
| Example 6 | 71-year-old female | hepatic cancer; metastasis to lung; end-stage cancer | 68% | 78 ng/mL | PR; metastasis disappear | |

### Examples 7 and 8: Combined administration of AHCC, PSK and shark cartilage

To the human cancer patients listed in Table 3, first, AHCC at a dosage of 3.0 to 6.0 g/day combined with shark cartilage at a dosage of 20 g/day were continuously orally administered. After three months no tumor regression could be diagnosed and the IL-12 level was not elevated.

Then, in addition to the continuous oral administration of AHCC at a dosage of 3.0 to 6.0 g/day combined with shark cartilage at a dosage of 20 g/day, PSK (Sankyo Co. Ltd., Krestin) at 3.0 g/day was further combinedly administered to the human cancer patients listed in Table 3. After additional PSK administration, the IL-12 level was elevated and at the same time, the PR effect of 50% or higher for the tumor was obtained.

It was shown that the concomitant use of shark cartilage had no effect on the NK activity and did not elevate the IL-12 level. Further, since the single administration of PSK at 3.0 g/day had not shown a regressive effect on the lung tumor, the observed regressive effect was thought to be achieved by the concomitant use of AHCC and PSK. It was suggested that PSK also promoted the IL-12 production.

**Table 3**

| | Human patient | Symptoms | NK activity | Serum IL-12 level | Treatment evaluation | CD4/CD8 ratio |
|---|---|---|---|---|---|---|
| Example 7 | 67-year-old male | left lung cancer; metastasis to right lung; metastasis to liver; endstage cancer | 32% | 240 ng/mL | PR; right lung metastasis disappear | 0.43 |
| Example 8 | 73-year-old female | right lung cancer; metastasis to left lung; metastasis to liver; endstage cancer | 29% | 340 ng/mL | PR; lung metatasis disappear | 0.43 |

### Examples 9 and 10: Concomitant use of OK-432, PSK and SPG (or Lentinan)

As shown in Table 4, the effect of multiple immunotherapy was tested in example 9, by subcutaneous administration of OK-432 (Chugai Pharmaceutical Co. Ltd., Picibanil) at a dosage of 5 KE/W, oral administration of PSK (Sankyo Co. Ltd., Krestin) at 3.0 g/day and further intramuscular injection of SPG (Kaken Pharmaceutical Co. Ltd., Sonifilan) at vial/W. In example 10, the effect of multiple immunotherapy was also tested in the same manner as in example 9, except that instead of SPG, Lentinan (Yamanouchi Pharmaceutical Co. Ltd., Lentinan) was intravenously injected at 400 mg/w.

In either example, a dramatic tumor regression was achieved. Further, the IL-12 level was high in either example. It was suggested that the anti-cancer effect obtained by the multiple immunotherapy may be due to the increased production of IL-12.

**Table 4**

| | Human patient | Symptoms | NK activity | Serum IL-12 level | Treatment evaluation | CD4/CD8 ratio |
|---|---|---|---|---|---|---|
| Example 9 | 23-year-old male | stomach cancer; carcinomatous peritonitis; end-stage cancer | 32% | 240 ng/mL | CR; complete regression | 0.72 |
| Example 10 | 72-year-old male | hepatic cancer; carcinomatous peritonitis; end-stage cancer | 40% | 260 ng/mL | PR; 3/4 of hepatic cancer disappearance | 0.32 |

For patients with progressed cancer or end-stage cancer, no example has been reported in which tumor regression or disappearance was achieved by administering BRM preparations to induce the IL-12 production. The examples of the present invention clarified that the observed effect was not only due to the NK activity but also to the induction of IL-12 and an increased level of killer T cells. It was also found that when the anti-cancer effect is to be enforced by the induction of IL-12, concomitant administration of shark cartilage that inhibits neovascularization was also effective.

Furthermore, it was found that additional PSK administration was effective in promoting the anti-cancer effect of AHCC. This effect was especially remarkable for lung cancer, hepatic cancer, stomach cancer, large intestine cancer, pancreatic carcinoma and kidney cancer.

It was found that IL-12 could be induced by concomitant administration of BRM preparations other than IL-12 or by concomitant administration of the three drugs OK-432, PSK and SPG (or Lentinan).

At present, even by making use of any treatment or technique of modern medicine, including surgery, administration of anti-cancer agents, radiotherapy and hormone therapy, little effect can be expected in the treatment of progressed cancer or end-stage cancer. Administration of an IL-12 inducer of the present invention or a medical composition comprising the same as the major component is highly effective in the practical use, showing high efficacy in the treatment of progressed cancer or end-stage cancer or in the improvement of QOL.

## Claims

1. A pharmaceutical composition comprising activated hemicellulose or derivatives thereof, optionally in combination with a pharmaceutically acceptable carrier.

2. The pharmaceutical composition of claim 1, which further comprises components of fungal mycelia.

3. The pharmaceutical composition of claim 1 or 2, which further comprises bacterial components of hemolytic streptococci.

4. Use of activated hemicellulose or derivatives thereof for the preparation of a pharmaceutical composition for inducing interleukin-12 (IL-12) or treating cancer.

5. Use of activated hemicellulose or derivatives thereof in combination with components of fungal mycelia for the preparation of a pharmaceutical composition for inducing IL-12 or treating cancer.

6. Use of activated hemicellulose or derivatives thereof in combination with components of fungal mycelia and bacterial components of hemolytic streptococci for the preparation of a pharmaceutical composition for inducing IL-12 or treating cancer.
